(19) Europäisches Patentamt
European Patent Office
Office européen des brevets

(11) **EP 4 108 283 A1**

(12) **EUROPEAN PATENT APPLICATION**
published in accordance with Art. 153(4) EPC

(43) Date of publication:
**28.12.2022 Bulletin 2022/52**

(21) Application number: **20919584.1**

(22) Date of filing: **24.11.2020**

(51) International Patent Classification (IPC):
***A61M 25/09*** (2006.01)

(52) Cooperative Patent Classification (CPC):
**A61M 25/09**

(86) International application number:
**PCT/JP2020/043585**

(87) International publication number:
**WO 2021/166354 (26.08.2021 Gazette 2021/34)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(30) Priority: **19.02.2020 JP 2020025801**

(71) Applicant: **Asahi Intecc Co., Ltd.
Seto-shi, Aichi 489-0071 (JP)**

(72) Inventors:
• **KOSUGI, Tomoki
Seto-shi, Aichi 489-0071 (JP)**
• **NOGUCHI, Naoki
Seto-shi, Aichi 489-0071 (JP)**
• **GOTO, Kenta
Seto-shi, Aichi 489-0071 (JP)**
• **NAKANISHI, Yuta
Seto-shi, Aichi 489-0071 (JP)**

(74) Representative: **Prinz & Partner mbB
Patent- und Rechtsanwälte
Rundfunkplatz 2
80335 München (DE)**

(54) **GUIDE WIRE**

(57) A guide wire includes a main body portion having an elongated outer shape and having a base and a linear marker alternately presented on an outer surface along an extending direction, and a resin layer having light permeability, covering the outer surface of the main body portion, and including a recessed and protruding portion where a recessed portion and a protruding portion are alternately formed along the extending direction of the main body portion, wherein in longitudinal cross-section along the extending direction of the main body portion, a width of the marker is wider than a width of the protruding portion.

FIG.1

## Description

### TECHNICAL FIELD

The disclosed embodiments relate to a guide wire.

### BACKGROUND ART

There is a conventionally known guide wire that includes a marker (visible marker) to observe the position and orientation of a distal end of the guide wire inserted into a living body lumen with an endoscopic camera. Also, there is a known guide wire having continuous recesses and protrusions formed on a surface to reduce frictional resistance between the guide wire and a body cavity wall when the guide wire is moved in a living body lumen (for example, Patent Literatures 1 to 4). For example, Patent Literature 1 discloses a guide wire that has protrusions formed on the surface and are covered with resin. Patent Literature 2 discloses a guide wire that has a raised-area forming layer functioning as a visible marker formed on the outer surface of the guide wire and has recesses and protrusion formed on the surface. Patent Literature 3 discloses a guide wire in which a resin film covers an outer periphery of a core wire having recesses and protrusions and the resin film has a spiral pattern formed therein. Patent Literature 4 discloses a guide wire in which a visible marker is provided on an outer surface of an inner layer.

### CITATION LIST

Patent Literature

> Patent Literature 1: JP 5619426
> Patent Literature 2: JP 5509276
> Patent Literature 3: WO2009/004876
> Patent Literature 4: JP 2003-275323 A

### SUMMARY OF INVENTION

### TECHNICAL PROBLEM

Even with the background arts described above, there is still room for improvements in the technique for improving the visibility of the marker of the guide wire when the marker is observed by an endoscopic camera. For example, when the guide wire having recesses and protrusions formed on the surface is irradiated with observation light in a living body lumen and observed by an endoscopic camera, the light reflected by the recesses and protrusions on the surface causes a light streak (bokeh circle) in the image (video) captured by the endoscopic camera, which may reduce the visibility of the marker.

The disclosed embodiments have been made to solve the above-described issue and has an object to provide the technique for improving the visibility of a marker provided in a guide wire.

### SOLUTION TO PROBLEM

The disclosed embodiments have been made to solve at least part of the above-described issue and may be implemented as the following aspects.

(1) According to an aspect of the disclosed embodiments, a guide wire is provided. The guide wire includes a main body portion having an elongated outer shape and having a base and a linear marker alternately presented on an outer surface along an extending direction, and a resin layer having light permeability, covering the outer surface of the main body portion, and including a recessed and protruding portion where a recessed portion and a protruding portion are alternately formed along the extending direction of the main body portion, wherein in longitudinal cross-section along the extending direction of the main body portion, a width of the marker is wider than a width of the protruding portion.
With this configuration, the width of the marker is wider than the width of the protruding portion of the resin layer in longitudinal cross-section along the extending direction of the main body portion, and therefore even when the light is reflected by the recessed and protruding portion of the resin layer covering the marker, a reduction in the visibility of the marker due to the light streak (bokeh circle) may be suppressed.
(2) In the guide wire according to the above aspect, a pitch of the marker may be different from a pitch of the protruding portion in the extending direction of the main body portion. With this configuration, as the position of the light streak with respect to the marker changes depending on each marker, the visibility of the marker may be further improved.
(3) In the guide wire according to the above aspect, the width of the marker may be equal to or less than twice the width of the protruding portion in longitudinal cross-section along the extending direction of the main body portion. With this configuration, the light streak is unlikely to appear over a plurality of protruding portions above the marker, and therefore a reduction in the visibility of the marker due to the light streak may be further suppressed.
(4) In the guide wire according to the above aspect, on the outer surface of the main body portion, a surface area of the marker in a range of 20 mm along the extending direction of the main body portion may be 35% or less of a surface area of the base. With this configuration, it is possible to further improve the visibility of the marker with respect to the base.
(5) In the guide wire according to the above aspect, on the outer surface of the main body portion, lightness of the marker may be lower than lightness of the base. With this configuration, it is possible to further improve the visibility of the marker with respect to the base.

(6) In the guide wire according to the above aspect, on the outer surface of the main body portion, a boundary portion between the base and the marker may be formed to be flat. With this configuration, it is possible to increase the flexibility in designing recesses and protrusions and to improve the slidability.

The disclosed embodiments may be implemented in various aspects and may be implemented in forms such as catheters, endoscopes, image generation devices, inspection devices, treatment systems, and methods for manufacturing guide wires.

BRIEF DESCRIPTION OF DRAWINGS

Fig. 1 is an explanatory diagram illustrating an example of an overall configuration of a guide wire according to a first embodiment.

Fig. 2 is an explanatory diagram illustrating an example of a cross-sectional configuration of the guide wire.

Fig. 3 is an enlarged explanatory diagram of a portion X in Fig. 2.

Fig. 4 is an explanatory diagram illustrating the state where the guide wire is used in a living body lumen.

Fig. 5 is an explanatory diagram illustrating the state of the guide wire observed by an endoscopic camera.

Fig. 6 is a diagram illustrating the state where the guide wire is irradiated with observation light.

Fig. 7 is an explanatory diagram illustrating the state where a guide wire according to a comparative example 1 is irradiated with the observation light.

Fig. 8 is an explanatory diagram illustrating the state where a guide wire according to a comparative example 2 is irradiated with the observation light.

Fig. 9 is a diagram illustrating the state where the guide wire and a combined device are used.

Fig. 10 is a diagram illustrating the state where a guide wire according to a comparative example 3 and the combined device are used.

Fig. 11 is an explanatory diagram illustrating an example of an overall configuration of a guide wire according to a second embodiment.

EMBODIMENTS OF THE INVENTION

<First Embodiment>

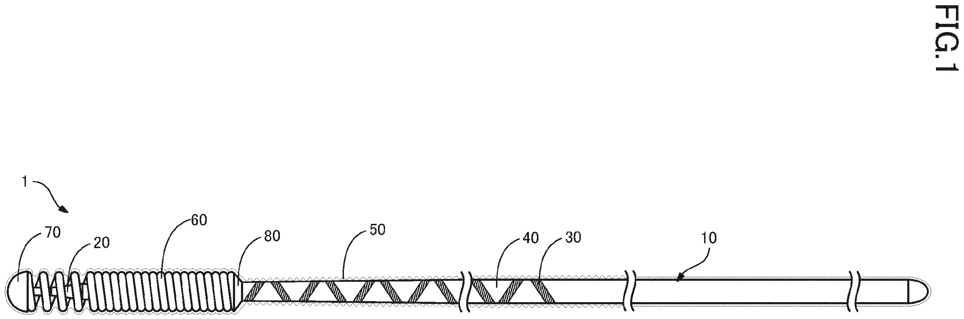

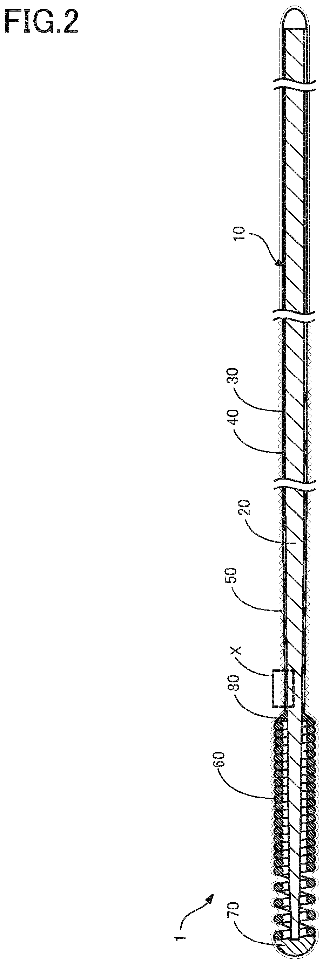

Fig. 1 is an explanatory diagram illustrating an example of an overall configuration of a guide wire 1 according to a first embodiment. Fig. 2 is an explanatory diagram illustrating an example of a cross-sectional configuration of the guide wire 1. Hereafter, the left side of Fig. 1 is referred to as "distal end side" of the guide wire 1 and each component, and the right side of Fig. 1 is referred to as "proximal end side" of the guide wire 1 and each component. The distal end side of the guide wire 1 is the side (distal side) inserted into the body, and the proximal end side of the guide wire 1 is the side (near side) operated by a manipulator such as a doctor. The right-and-left direction in Fig. 1 is also referred to as "extending direction" or "axial direction" of the guide wire 1 and each component. Fig. 2 illustrates a longitudinal cross-section surface of the guide wire 1 along the extending direction. The guide wire 1 is a medical device that is used when a catheter is inserted into a blood vessel or digestive organ and that includes a main body portion 10, a resin layer 50, a coil body 60, a distal end joint part 70, and a proximal end side joint part 80.

The main body portion 10 has an elongated shape and includes a core shaft 20, a marker 30, and a base 40. The resin layer 50 is formed on an outer surface of the main body portion 10, and the coil body 60 is fixed at the distal end of the main body portion 10.

The core shaft 20 is an elongated member configured to have an outer diameter that decreases (tapered) from the proximal end side toward the distal end side. The core shaft 20 may be made of a material such as stainless alloy (SUS302, SUS304, SUS316, etc.), superelastic alloy such as Ni-Ti alloy, piano wire, nickel-chromium based alloy, cobalt alloy, and tungsten. The core shaft 20 may be made of a known material other than the materials described above. The length of the core shaft 20 is not particularly limited but may be for example in the range from 1000 mm to 5000 mm. Also, the outer diameter of the core shaft 20 is not particularly limited but may be for example in the range from 0.1 mm to 1.0 mm. The distal end joint part 70 is formed at the distal end of the core shaft 20. The proximal end side joint part 80 is formed on the outer periphery of the core shaft 20 on the proximal end side by a predetermined distance away from the distal end. The coil body 60 is provided between the distal end joint part 70 and the proximal end side joint part 80.

The base (base layer) 40 is a resin formed on the outer surface of the core shaft 20 to cover the outer periphery of the core shaft 20 on the proximal end side with respect to the position where the proximal end side joint part 80 is formed. The base 40 may be made of, for example, PAI (polyamide imide), PTFE (polytetrafluoroethylene), PVDF (polyvinylidene fluoride), PFA (perfluoroalkoxy alkane), FEP (perfluoroethylene propylene), ETFE (ethylene tetrafluoroethylene), PE (polyethylene), PP (polypropylene), etc. The type of resin included in the base 40 is not limited to the above and may be any resin. The base 40 may cover not only the outer periphery of the core shaft 20 on the proximal end side with respect to the proximal end side joint part 80 but also the outer periphery on the distal end side. The base 40 is substantially white, and the Munsell lightness is in the range from 7 to 10.

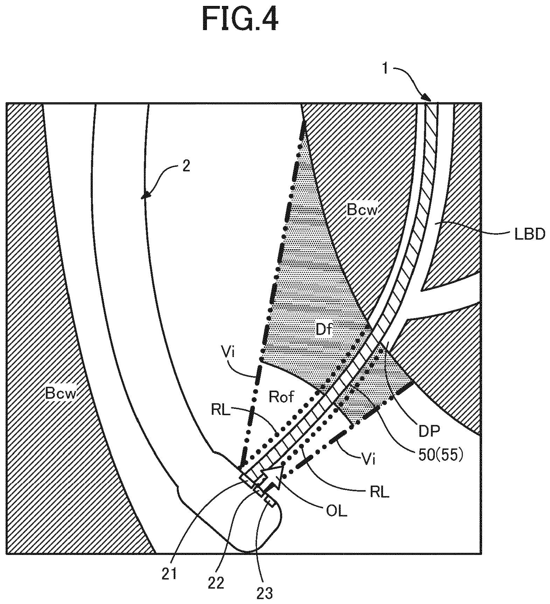

The marker 30 is a linear portion formed at part of the base 40 and is configured to be visually distinguishable from other portions of the base 40. When the guide wire 1 is observed from outside (observed through an image sensor 23 of an endoscope 2 described in Fig. 4 below), the marker 30 appears as a linear pattern on the base 40, and the manipulator observes changes in the orientation and position of the pattern while operating the guide wire 1 so as to confirm the operation of pushing, pulling, and rotating the guide wire 1. The marker 30 is formed in a section (marker presentation section) of the base 40 from the distal end in contact with the proximal end side joint part 80 toward the rear end side by a predetermined distance. Specifically, it is formed on a portion that protrudes from the distal end of the endoscope 2 and that is observed through the image sensor 23 (see Fig. 4). The length of the portion (marker presentation section) of the base 40 where the marker 30 is formed is not particularly limited but may be for example in the range from 100 mm to 500 mm. In the main body portion 10, the base 40 and the linear marker 30 are alternately presented along the extending direction in the marker presentation section.

The marker 30 is drawn as a linear pattern having a constant width by impregnating part of the base 40 with a pigment and partially changing the color (at least one of hue, lightness, and saturation) of the base 40. Specifically, here, the marker 30 is not protruded or recessed outward in the radial direction of the main body portion 10 with respect to other portions of the base 40, and a boundary portion between the base 40 and the marker 30 is formed to be flat. The marker 30 is substantially black, and the Munsell lightness is in the range from 0 to 7. The Munsell lightness of the marker 30 is preferably in the range from 0 to 2. The marker 30 and the base 40 have different lightness, and the lightness of the marker 30 is lower than that of the base 40. The color of the base 40 is relatively close to white, and the color of the marker 30 is relatively close to black.

The marker 30 is formed to have a wave-shaped pattern in the net diagram of the base 40 having a hollow cylindrical shape covering the outer periphery of the core shaft 20. Specifically, the wave pattern may be formed such that, while the core shaft 20 having the base 40 formed thereon is rotated forward and backward around the rotation axis in the extending direction (axial direction) by a predetermined angle (e.g., 180°) to right and left, the pigment is dropped toward the base 40 and moved along the extending direction. The marker 30 may be formed in a spiral shape on the base 40 having a hollow cylindrical shape. Specifically, the spiral pattern may be formed such that, while the core shaft 20 having the base 40 formed thereon is rotated in one direction around the rotation axis in the extending direction (axial direction), the pigment is dropped toward the base 40 and moved along the extending direction.

The resin layer 50 is a light-transmitting resin film, here a transparent film, formed on the marker 30 and the base 40. Fluorine resin such as PTFE (polytetrafluoroethylene) and PFA (perfluoroalkoxy alkane), silicone resin, polyurethane, polyethylene, polyvinyl chloride, polyester, polypropylene, polyamide, polystyrene, and the like, may be used for the resin layer 50. An outer surface of the resin layer 50 includes a recessed and protruding portion where a recessed portion and a protruding portion are alternately formed along the extending direction of the main body portion 10 (the core shaft 20). As the recessed and protruding portion is formed on the entire guide wire 1 in the circumferential direction, the outer diameter of the guide wire 1 changes due to the recessed and protruding portion. Specifically, the protruding portion of the resin layer 50 enlarges the outer diameter of the guide wire 1, and the recessed portion of the resin layer 50 reduces the outer diameter of the guide wire 1. The recessed and protruding portion of the resin layer 50 will be described below in detail.

The distal end joint part 70 is made of metal solder such as silver solder, gold solder, zinc, Sn-Ag alloy, and Au-Sn alloy, and the distal end of the coil body 60 and the distal end of the core shaft 20 are fixed to each other with the metal solder. The distal end joint part 70 may be made of an adhesive such as epoxy adhesive, and the distal end of the coil body 60 and the distal end of the core shaft 20 may be fixed to each other with the adhesive.

The coil body 60 includes one or more coils and is wound around the core shaft 20 to cover the outer periphery of the core shaft 20 on the distal end side. Here, the coil body 60 is wound around a small diameter portion and part of a tapered portion of the core shaft 20 on the distal end side. The coil included in the coil body 60 may be a single coil formed in a hollow cylindrical shape by winding a single wire having a circular cross-section surface in a spiral manner or may be a hollow twisted wire coil formed in a hollow cylindrical shape by a twisted wire that is obtained by twisting a plurality of wires. The coil body 60 may be configured by combining a single coil and a hollow twisted wire coil. The coil body 60 may be made of, for example, stainless alloy (SUS302, SUS304, SUS316, etc.), superelastic alloy such as Ni-Ti alloy, piano wire, nickel-chromium based alloy, cobalt alloy, radiotransparent alloy such as tungsten, gold, platinum, tungsten, and radiopaque alloy such as alloy containing these elements (e.g., platinumnickel alloy). The coil body 60 may be made of a known material other than the materials described above. The length of the coil body 60 is not particularly limited but may be for example from 10 mm to 100 mm. The outer diameter of the coil body 60 is not particularly limited but may be for example in the range from 0.1 mm to 1.0 mm and is configured to be constant from the distal end to the proximal end. The coil body 60 may include a sparsely wound portion and a tightly wound portion with different coil pitches.

The distal end of the coil body 60 is joined to the distal end of the core shaft 20 by the distal end joint part 70. The proximal end of the coil body 60 is joined to the core shaft 20 by the proximal end side joint part 80. The coil body 60 is fixed to the core shaft 20 by the distal end joint part 70 and the proximal end side joint part 80. The proximal end side joint part 80 is a circular (ring-shaped) portion provided on the outer periphery of the core shaft 20 and here is made of the same material as that of the distal end joint part 70. The proximal end side joint part 80 may be made of a different material from that of the distal end joint part 70.

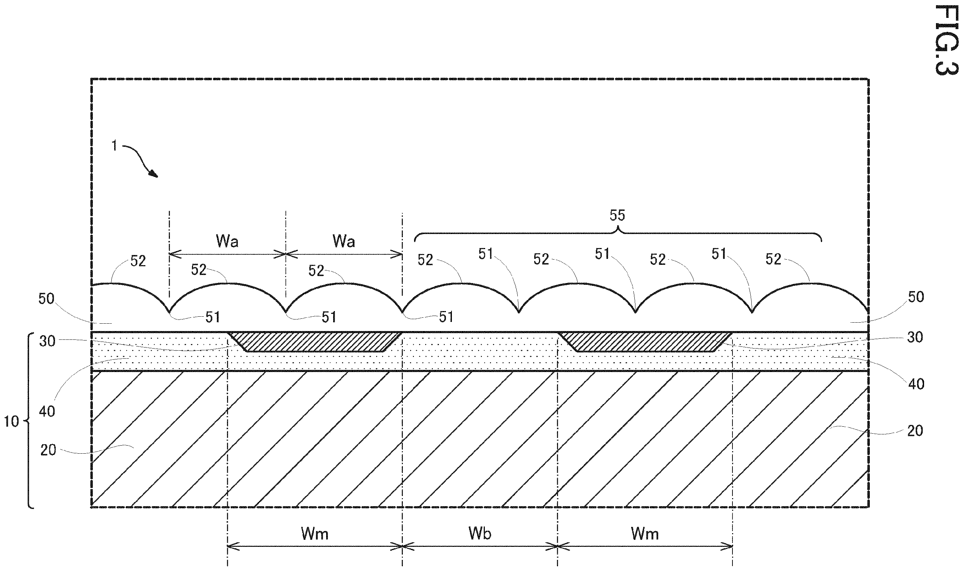

Fig. 3 is an enlarged explanatory diagram of a portion X in Fig. 2. Here, the detailed configurations of the marker 30, the base 40, and the resin layer 50 in the marker presentation section will be described. The resin layer 50 includes a recessed and protruding portion 55 where a recessed portion 51 and a protruding portion 52 alternately continue along the extending direction (the right-and-left direction in Fig. 3) of the main body portion 10. Here, Wm is the width of the marker 30 in the extending direction of the main body portion 10 (in longitudinal cross-section of the main body portion 10), Wb is the width of the base 40 in the extending direction of the main body portion 10 (in longitudinal cross-section of the main body portion 10), and Wa is the width of the protruding portion 52 in the extending direction of the main body portion 10 (in longitudinal cross-section of the main body portion 10). The width Wb of the base 40 is equal to the distance from one of the adjacent markers 30 to the other one (the interval between the markers). The width Wm of the marker 30 here is the average value of the distance from one end of each of the markers 30, which are arranged in the extending direction of the main body portion 10, to the other end in longitudinal cross-section of the main body portion 10. The width Wb of the base 40 is the average value of the distance from the end of one of the adjacent markers 30 to the end of the other marker 30 in longitudinal cross-section of the main body portion 10. The width Wa of the protruding portion 52 is the average value of the distance from one end of each of the protruding portions 52, which are arranged in the extending direction of the main body portion 10, to the other end in longitudinal cross-section of the main body portion 10.

The marker 30, the base 40, and the resin layer 50 are configured such that, in the extending direction of the main body portion 10, a pitch Pm of the marker 30 is different from a pitch Pp of the protruding portion 52 of the resin layer 50 (Pm ≠ Pp). The pitch of the marker 30 here is the average value of the distance between the center positions of the markers 30 arranged in the extending direction of the main body portion 10 in longitudinal cross-section of the main body portion 10. The pitch of the protruding portion 52 of the resin layer 50 is the average value of the distance between the center positions of the protruding portions 52 arranged in the extending direction of the main body portion 10 in longitudinal cross-section of the main body portion 10. The pitch Pm of the marker 30 is equal to the sum of the width Wm of the marker 30 and the width Wb of the base 40 (Pm = Wm + Wb). The pitch Pp of the protruding portion 52 is equal to the width Wa of the protruding portion 52 (Pp = Wa). Accordingly, the marker 30, the base 40, and the resin layer 50 satisfy the following Equation (1).

$$Wm + Wb \neq Wa ...(1)$$

Thus, the position where a light streak (bokeh circle) appears on the marker 30 changes depending on each of the markers 30, which may improve the visibility of the marker 30. The reason for this will be described below.

In the marker 30, the base 40, and the resin layer 50, the width Wm of the marker 30 is larger than the width Wa of the protruding portion 52 and is smaller than twice the width Wa of the protruding portion 52. Specifically, the marker 30, the base 40, and the resin layer 50 satisfy the following Equation (2).

$$Wa < Wm < 2 \times Wa ...(2)$$

Accordingly, even when the light is reflected by the recessed and protruding portion 55 of the resin layer 50 covering the marker 30, a reduction in the visibility of the marker 30 due to a light streak (bokeh circle) may be suppressed. The reason for this will be described below.

In the marker 30 and the base 40, a surface area Am of the marker 30 is 35% or less of a surface area Ab of the base 40 (Am ≤ 0.35 × Ab) in the marker presentation section. That is, the marker 30 and the base 40 satisfy the following Equation (3).

$$Wm \leq 0.35 \times Wb ...(3)$$

This may improve the visibility of the marker 30 with respect to the base 40. Specifically, when the surface area Am of the marker 30 is larger than 35% of the surface area Ab of the base 40, the percentage of the marker 30 occupied in the captured image increases, which makes it difficult to determine the number of the markers 30 when a light streak (bokeh circle) appears and causes a reduction in the visibility of the marker.

The width Wm of the marker 30, the width Wb of the base 40, and the width Wa of the protruding portion 52 described above are the average value of the widths of the markers 30, the average value of the widths of the bases 40, and the average value of the widths of the protruding portions 52 included in the range of 20 mm along the extending direction of the main body portion 10 in the marker presentation section of the main body portion 10. Specifically, the width Wm is obtained by, in longitudinal cross-section of the main body portion 10 in the marker presentation section, dividing the total of the widths of the markers 30 included in the arbitrary range of 20 mm by the number of the markers 30 included in the range. The width Wb is obtained by dividing the total of the widths of the bases 40 between the markers 30 included in the range by the number of the bases between the markers 30 included in the range. The width Wa is obtained by dividing the total of the widths of the protruding portions 52 included in the range by the number of the protruding portions 52 included in the range.

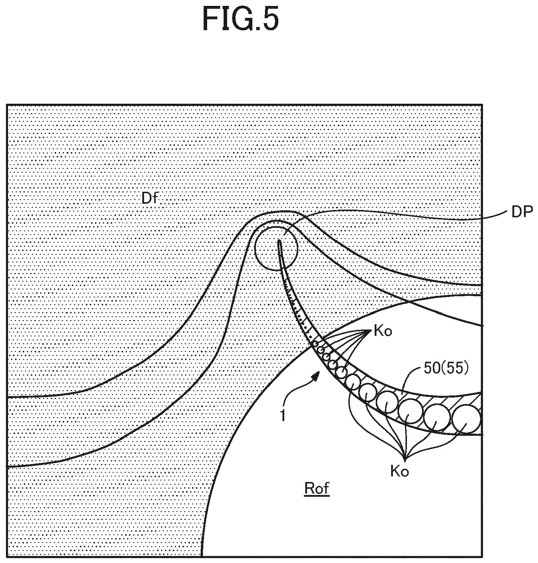

Fig. 4 is an explanatory diagram illustrating the state where the guide wire 1 is used in a living body lumen. Fig. 5 is an explanatory diagram illustrating the state of the guide wire 1 that is observed by an endoscopic camera in a living body lumen. Fig. 4 illustrates the state where the guide wire 1 is protruded from the distal end of the endoscope (posterior obliqueviewing endoscope) 2 delivered into the duodenum and the distal end side of the guide wire 1 is delivered from a duodenal papilla DP to a distal bile duct LBD. In Fig. 4, the finely shaded portion represents a body cavity wall Bcw of the duodenum. The thick double-dashed line represents a field of view Vi of the endoscopic camera (the image sensor 23). The fine dot portion inside the field of view Vi represents a depth of field Df, and the blank portion represents an out-of-depth of field region (out-of-focus region) Rof outside the depth of field Df. The depth of field Df indicates the region that appears to be in focus in the endoscopic camera, and the out-of-depth of field region Rof indicates the region that is out of focus near the endoscopic camera. Fig. 5 illustrates a captured image (captured video) with the endoscope viewpoint captured by the endoscopic camera (the image sensor 23), an object located in the out-of-depth of field region Rof is displayed in the lower right of the image (video), and an object located in the depth of field Df is displayed in the other areas.

The endoscope 2 is an electronic endoscope with what is called a simultaneous method as an imaging method and includes an opening 21, a light emitting portion 22, and the image sensor 23 at the distal end. The opening 21 communicates with a lumen (not illustrated) inside the endoscope 2, and a distal end portion of the guide wire 1 in the lumen may protrude through the opening 21. The opening 21 is configured such that the protruding direction of the distal end portion of the guide wire 1 intersects the extending direction of the endoscope 2. The light emitting portion 22 is provided at the distal end of the endoscope 2 to emit white light (observation light) OL from a white light source such as a halogen lamp, xenon lamp, LED lamp, or laser. The image sensor 23 is a color imaging device with a color filter provided on the front surface of the CCD and functions as an endoscopic camera. The endoscope 2 may be an electronic endoscope with what is called a frame sequential method as an imaging method.

As illustrated in Fig. 4, when the observation light OL is emitted from the light emitting portion 22 toward the guide wire 1 while the guide wire 1 protrudes from the opening 21, reflected light RL, which is reflection of the observation light OL, occurs on the surface of the guide wire 1. In this state, when the guide wire 1 is captured by the image sensor 23 (endoscopic camera), part of the reflected light RL, reflected by the recessed and protruding portion 55 of the resin layer 50, appears as a light streak (reflected light streak) Ko in the out-of-depth of field region Rof as illustrated in Fig. 5. The light streak Ko is what is called "bokeh circle" and is a blurry light point that appears in the captured image (captured video) captured by the endoscopic camera. The recessed and protruding portion 55 of the resin layer 50 in the depth of field Df is in focus, and therefore substantially none of the light streak (bokeh circle) Ko appears in a portion of the depth of field Df.

The reason why the light streak Ko appears in the out-of-depth of field region Rof will be described. When the surface of the guide wire 1 is irradiated with the observation light (irradiation light) OL from the endoscope 2, the recessed and protruding portion 55 on the surface of the resin layer 50 reflects the observation light OL and acts as a light source that emits the reflected light RL. In the out-of-depth of field region Rof, the reflected light RL of the recessed and protruding portion 55 is optically "a light source outside the depth of field" and therefore appears as the light streak (reflected light streak) Ko in the captured image (captured video) captured by the endoscopic camera. The light streak Ko appearing in the recessed and protruding portion 55 of the resin layer 50 causes what is called "flicker" and reduces the visibility of the marker 30 in the captured image.

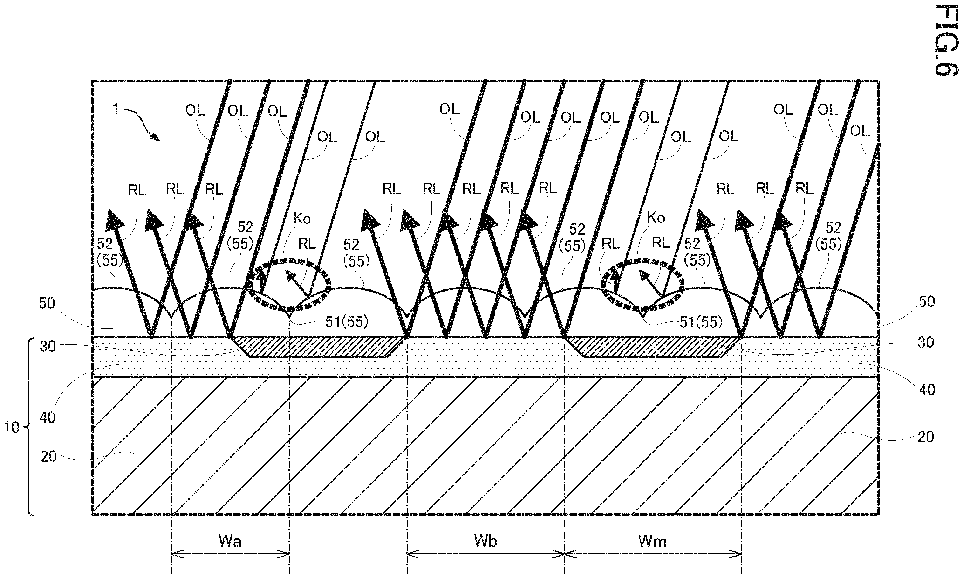

Fig. 6 is an explanatory diagram illustrating the state where the guide wire 1 is irradiated with the observation light OL. Fig. 6 illustrates the same portion as that in Fig. 3. As described above, the imaging method of the endoscope 2 is what is called a simultaneous method, and therefore the light emitting portion 22 of the endoscope 2 emits the white observation light OL of a xenon lamp. When the outer surface of the guide wire 1 is irradiated with the white observation light OL, both the surface of the resin layer 50 and the surface of the base 40 cause reflection. In Fig. 6, the observation light OL emitted to the surface of the resin layer 50 and the reflected light RL are illustrated in thin lines, and the observation light OL emitted to the surface of the base 40 and the reflected light RL are illustrated in bold lines. As the marker 30 is black, the surface of the marker 30 causes substantially no reflection of the observation light OL.

As the observation light OL emitted from the light emitting portion 22 and the base 40 are both white, the reflected light RL reflected by the base 40 is a relatively intense white light. Therefore, in the resin layer 50 covering both the surface of the marker 30 and the surface of the base 40, the recessed and protruding portion 55 of the resin layer 50 covering the surface of the base 40 is irradiated with the intense light (the reflected light RL) from a background light source (the base 40). Therefore, in the resin layer 50 covering the surface of the base 40, the light streak Ko is canceled out by the white reflected light RL from the base 40. In other words, in the resin layer 50 covering both the surface of the marker 30 and the surface of the base 40, the light streak Ko appears in the recessed and protruding portion 55 of the resin layer 50 covering the surface of marker 30, and the light streak Ko is canceled out to make no appearance in the recessed and protruding portion 55 of the resin layer 50 covering the surface of the base 40.

In the recessed and protruding portion 55 of the resin layer 50, the reflected light RL, reflected by the resin layer 50, gathers near the recessed portion 51, and therefore the light streak Ko appears near the recessed portion 51 of the resin layer 50 covering the surface of the marker 30. In other words, the light streak Ko appears near the trough of the recessed and protruding portion 55 in the resin layer 50 covering the marker 30. In the guide wire 1 according to the present embodiment, as represented by Equation (2) above, the width Wm of the marker 30 is wider than the width Wa of the protruding portion 52, and therefore the width Wm of the marker 30 is wider than the width of the light streak Ko appearing near the trough of the recessed and protruding portion 55. Therefore, even when the visibility of part of the marker 30 is reduced due to the light streak Ko appearing in the resin layer 50 covering the marker 30, there is a portion that is not located under the light streak Ko in the identical marker 30, and therefore the marker 30 is visually recognizable in the image (captured video). In the left marker 30 of the two markers 30 in Fig. 6, the visibility of the left side of the marker 30 is reduced by the light streak Ko, but the right side of the marker 30 is not overlapped with the light streak Ko so as to be visible. In the right marker 30 of the two markers 30 in Fig. 6, the visibility is reduced near the center of the marker 30 due to the light streak Ko, but the right and left edges of the marker 30 are not overlapped with the light streak Ko so as to be visible.

As represented by Equation (1) above, the guide wire 1 according to the present embodiment is configured such that the pitch (= Wm + Wb) of the marker 30 is different from the pitch (= Wa) of the protruding portion 52 of the resin layer 50. Accordingly, the position where the light streak Ko appears on the marker 30 changes depending on each of the markers 30, which makes it easy to determine the actual width of the marker 30 and makes it possible to improve the visibility of the marker 30. In the guide wire 1 according to the present embodiment, as represented by Equation (3) above, the surface area of the marker 30 is 35% or less of the surface area of the base 40. Specifically, the width Wm of the marker 30 is smaller than 0.35 times the width Wb of the base 40. Thus, when the light streak Ko appears over the marker 30, it is easy to distinguish whether the marker 30 on both sides of the light streak Ko is the one identical marker or the two different markers 30.

The imaging method of the endoscope 2 is what is called a simultaneous method in the description but may also be a frame sequential method. When the imaging method of the endoscope 2 is a frame sequential method, the light emitting portion 22 uses an RGB rotary filter to disperse and emit the white light from the xenon lamp. The image sensor 23 is a monochromatic CCD image sensor. Even in this case, the observation light OL is perceived as a white light by the human eye that checks the captured image (captured video) and is perceived as the same color as that of the base 40. Therefore, as described above, in the resin layer 50 covering both the surface of the marker 30 and the surface of the base 40, the light streak Ko appears in the recessed and protruding portion 55 of the resin layer 50 covering the surface of the marker 30, and the light streak Ko is canceled out to make no appearance in the recessed and protruding portion 55 of the resin layer 50 covering the surface of the base 40. Thus, with the guide wire 1, the same effect may be obtained regardless of the imaging method of the endoscope 2.

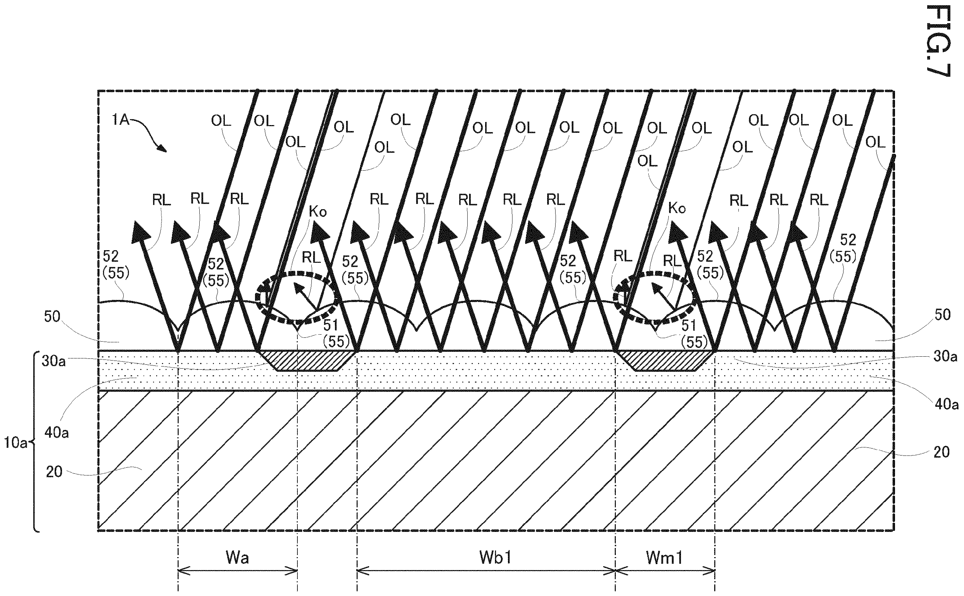

Fig. 7 is an explanatory diagram illustrating the state where a guide wire 1A according to a comparative example 1 is irradiated with the observation light OL. In the guide wire 1A according to the comparative example 1, as compared to the guide wire 1 (Fig. 6) according to the first embodiment, a width Wm1 of a marker 30a according to the comparative example 1 is smaller than the width Wm of the marker 30 (Fig. 6) according to the first embodiment (Wm1 < Wm). A width Wb1 of a base 40a according to the comparative example 1 is larger than the width Wb of the base 40 according to the first embodiment (Wb1 > Wb). The other configurations are the same as those of the guide wire 1 according to the first embodiment. That is, the width Wa of the protruding portion 52 of the resin layer 50 is identical.

The guide wire 1A according to the comparative example 1 does not satisfy Equation (2) above, and the width Wm1 of the marker 30a is smaller than the width Wa of the protruding portion 52 (Wm1 < Wa). Therefore, the width of the light streak Ko appearing near the trough of the recessed and protruding portion 55 is substantially identical to the width Wma of the marker 30a. That is, as illustrated in Fig. 7, the visibility of the entire marker 30a is reduced due to the light streak Ko appearing in the resin layer 50 covering the marker 30a.

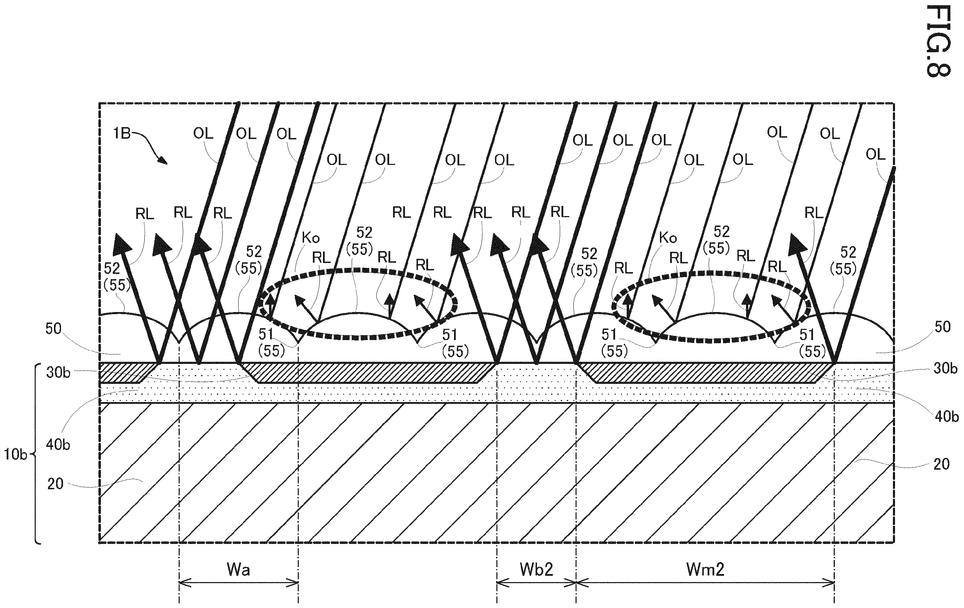

Fig. 8 is a diagram illustrating the state where a guide wire 1B according to a comparative example 2 is irradiated with the observation light. In the guide wire 1B according to the comparative example 2, as compared to the guide wire 1 (Fig. 6) according to the first embodiment, a width Wm2 of a marker 30b according to the comparative example 2 is larger than the width Wm of the marker 30 (Fig. 6) according to the first embodiment (Wm2 > Wm). A width Wb2 of a base 40b according to the comparative example 2 is smaller than the width Wb of the base 40 according to the first embodiment (Wb2 < Wb). The other configurations are the same as those of the guide wire 1 according to the first embodiment. That is, the width Wa of the protruding portion 52 of the resin layer 50 is identical.

The guide wire 1B according to the comparative example 2 does not satisfy Equation (2) above, and the width Wm2 of the marker 30b is larger than twice the width Wa of the protruding portion 52 ($Wm2 > 2 \times Wa$). Therefore, the recessed portions 51 of the resin layer 50 are located above the one marker 30b, and the light streak Ko appears in each of the recessed portions 51. As the adjacent light streaks Ko join together and seem to be the one large light streak Ko, the visibility of the marker 30b between the two light streaks Ko is largely reduced. Therefore, as illustrated in Fig. 8, the entire visibility of the marker 30b near the center is reduced due to the large light streak Ko joining the light streaks Ko, and only the right and left edges of the marker 30b are not overlapped with the light streak Ko so as to be visible. As described above, when the width Wm2 of the marker 30b is larger than twice the width Wa of the protruding portion 52, it is difficult to suppress a reduction of the visibility due to the light streak Ko regardless of the width of the marker 30b. Conversely, when the marker 30b has a large width, it is difficult to perceive the entire marker 30b in the captured image, which results in a reduction of the visibility.

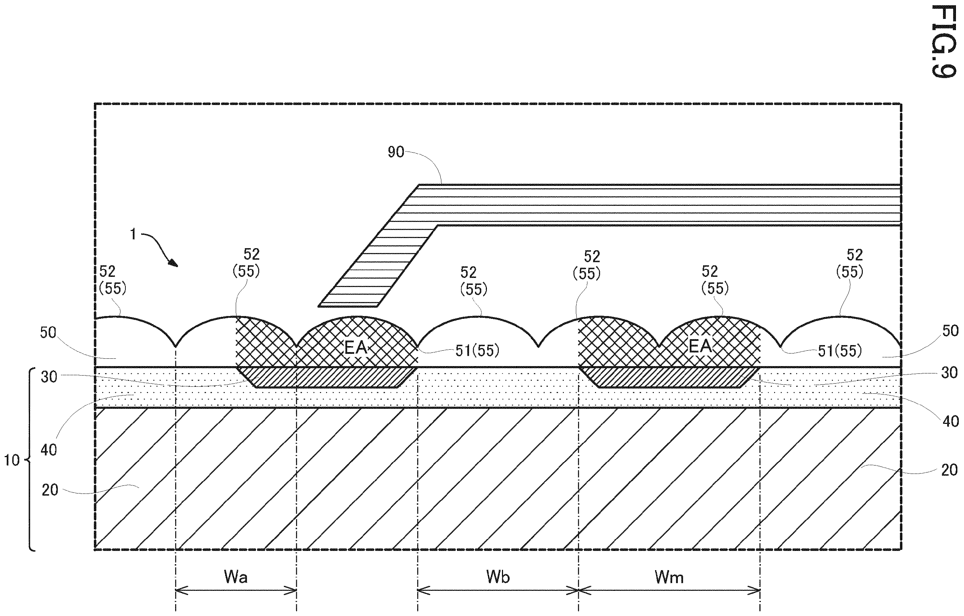

Fig. 9 is an explanatory diagram illustrating the state where the guide wire 1 and a combined device 90 are used. Fig. 9 illustrates the same portion as that in Fig. 3. As described above, in the marker 30, the base 40, and the resin layer 50, the width Wm of the marker 30 is larger than the width Wa of the protruding portion 52 ($Wa < Wm$). Therefore, in the resin layer 50, a recess and protrusion of the recessed and protruding portion 55 are positioned on a surface of a portion EA located above the marker 30. As the marker 30 contains a large amount of pigment, the marker 30 has a low affinity and a relatively low adhesion for the resin layer 50. Therefore, when the combined device 90 comes into contact with the surface of the resin layer 50, the combined device 90 is caught due to the frictional force between the combined device 90 and the resin layer 50, which may cause the resin layer 50 to be separated from the marker 30. With the guide wire 1 according to the present embodiment, the formation of recesses and protrusions reduces the frictional force between the combined device 90 and the resin layer 50 (improves the slidability) when the combined device 90 comes into contact with the resin layer 50 and therefore may suppress the separation of the resin layer 50.

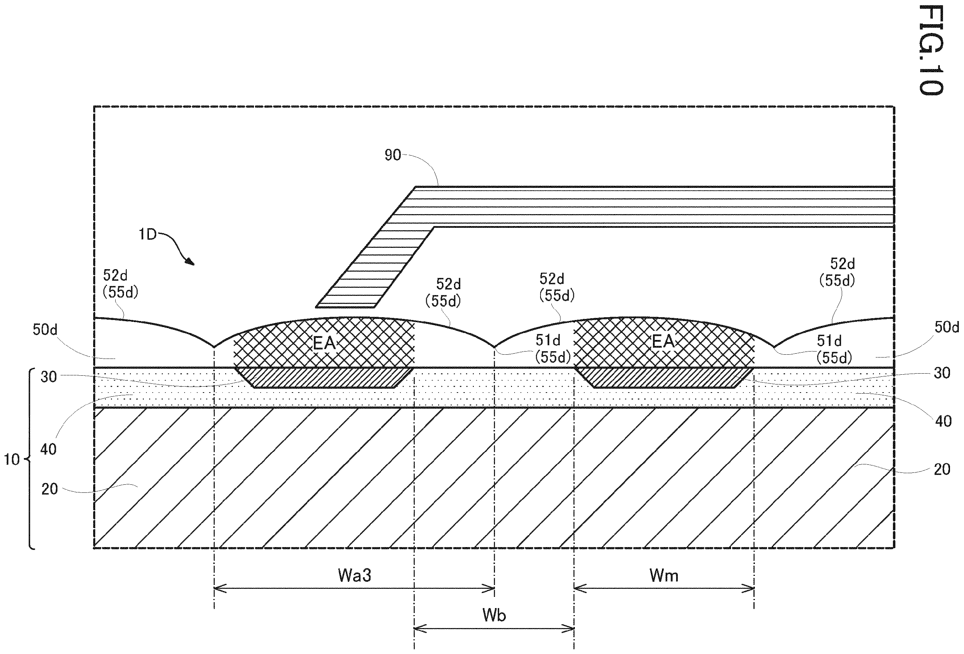

Fig. 10 is an explanatory diagram illustrating the state where a guide wire 1D according to a comparative example 3 and the combined device 90 are used. In the guide wire 1D according to the comparative example 3, as compared to the guide wire 1 (Fig. 6) according to the first embodiment, the resin layer 50 has a different configuration. Specifically, in a resin layer 50d according to the comparative example 3, a width Wa3 of a protruding portion 52d is larger than the width Wm of the marker 30 ($Wa3 > Wm$). The other configurations are the same as those of the guide wire 1 according to the first embodiment. That is, the width Wm of the marker 30a and the width Wb of the base 40 are identical.

In the guide wire 1D according to the comparative example 3, the width Wa3 of the protruding portion 52d is larger than the width Wm of the marker 30 ($Wa3 > Wm$), and therefore, in the resin layer 50d, there are no protrusions and recesses or a few protrusions and recesses of a recessed and protruding portion 55d on the surface of the portion EA located above the marker 30. Therefore, the surface of the portion EA of the resin layer 50d is relatively flat. Accordingly, when the combined device 90 comes into contact with the surface of the resin layer 50d, the combined device 90 is likely to be caught due to the frictional force between the combined device 90 and the resin layer 50d, and the resin layer 50d is likely to be separated from the marker 30.

With the guide wire 1 according to the present embodiment described above, as illustrated in Fig. 6, the width Wm of the marker 30 is wider than the width Wa of the protruding portion 52 in longitudinal cross-section along the extending direction of the main body portion 10, and therefore, even when the light is reflected by the recessed and protruding portion 55 of the resin layer 50 covering the marker 30, a reduction in the visibility of the marker 30 due to the light streak (bokeh circle) Ko may be suppressed. The guide wire 1 according to the present embodiment, which is the guide wire having the resin layer 50 of the recessed and protruding portion 55 formed on the surface, is to solve the novel issue of a reduction in the visibility of the marker 30 due to the light streak Ko occurring in the recessed and protruding portion 55 during imaging, and such an issue is not disclosed or indicated in Patent Literatures 1 to 4.

With the guide wire 1 according to the present embodiment, the formation of the recessed and protruding portion 55 on the surface of the resin layer 50 may reduce the contact area with the body cavity wall, reduce the friction during sliding, and improve the slidability. The recess and protrusion on the surface of the resin layer 50 may improve the slidability with the combined device 90.

With the guide wire 1 according to the present embodiment, the boundary portion between the base 40 and the marker 30 is formed to be flat. Thus, compared to the case where the marker 30 protrudes, the height (position) of the recessed portion 51 and the protruding portion 52 of the recessed and protruding portion 55 of the resin layer 50 may be more uniform. The pitch of the protruding portion 52 and the pitch of the marker 30 may be easily different. Thus, it is possible to increase the flexibility in designing recesses and protrusions and to improve the slidability. As the boundary portion between the base 40 and the marker 30 is flat, the marker 30 is embedded in the base 40, which may increase the contact area between the base 40 and the marker 30 and may enhance the separation resistance.

With the guide wire 1 according to the present embodiment, as the color of the base 40 is close to white, the light streak Ko may be canceled out in the resin layer 50 above the base 40. With the guide wire 1 according to the present embodiment, as there is a high contrast between the marker 30 and the base 40, the visibility of the marker 30 may be improved.

<Second Embodiment>

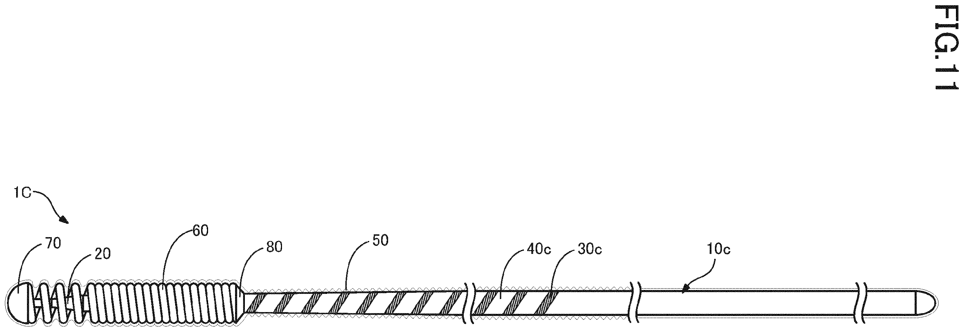

Fig. 11 is an explanatory diagram illustrating an example of an overall configuration of a guide wire 1C according to a second embodiment. In the guide wire 1C according to the second embodiment, as compared to the guide wire 1 (Fig. 1) according to the first embodiment, a marker 30c has a different presentation form. The other configurations are the same as those of the guide wire 1 according to the first embodiment, and therefore the description is omitted. The marker 30c according to the second embodiment has a spiral pattern having a constant width. Therefore, it is formed to have a pattern in which a plurality of oblique lines is arranged in the axial direction in the net diagram of a base 40c having a hollow cylindrical shape covering the outer periphery of the core shaft 20. The spiral pattern may be formed such that, while the core shaft 20 having the base 40c formed thereon is rotated in one direction around the rotation axis in the extending direction (axial direction), the pigment is dropped toward the base 40c and moved along the extending direction. The marker 30c has a constant pitch.

As the marker 30c, the base 40c, and the resin layer 50 satisfy Equation (1) above, the position where the light streak (bokeh circle) appears on the marker 30c may be changed depending on each of the markers 30c. Thus, the visibility of the marker 30 may be improved. As the marker 30c, the base 40c, and the resin layer 50 satisfy Equation (2) above, a reduction in the visibility of the marker 30 due to the light streak (bokeh circle) may be suppressed even when the light is reflected by the recessed and protruding portion 55 of the resin layer 50 covering the marker 30c. As the marker 30c and the base 40c satisfy Equation (3) above, the visibility of the marker 30c with respect to the base 40c may be improved.

<Modifications of Present Embodiment>

The disclosed embodiments are not limited to the above-described embodiments and may be implemented in various aspects without departing from the spirit thereof, and for example the following modifications are possible.

[Modification 1]

The markers 30 and 30c according to the first embodiment and the second embodiment have a constant pitch. However, the pitch of the markers 30 and 30c may be non-constant. The markers 30 and 30c have a constant width. However, the width of the markers 30 and 30c may vary halfway.

[Modification 2]

The markers 30 and 30c according to the first embodiment and the second embodiment are presented on part (marker presentation section) of the bases 40 and 40c. However, the markers 30 and 30c may be formed on the entire bases 40 and 40c. The markers 30 and 30c have a single pattern (pattern) drawn. However, the markers 30 and 30c may have a plurality of types of patterns. In this case, the markers 30 and 30c may have the form of a continuously changing pattern or may have a different pattern for each predetermined section. The markers 30 and 30c may also be disconnected in the middle of the bases 40 and 40c and drawn in a plurality of areas. For example, in the markers 30 and 30c, a plurality of types of patterns may be drawn in a plurality of areas with a predetermined interval, or a circular pattern may be continuously drawn at an equal interval.

[Modification 3]

In the recessed and protruding portion 55 of the resin layer 50 according to the first embodiment and the second embodiment, the protruding portion 52 has a constant pitch. However, the pitch of the protruding portion 52 may be non-constant. The resin layer 50 may partially include a flat portion instead of the recessed and protruding portion 55.

[Modification 4]

According to the first embodiment and the second embodiment, the markers 30 and 30c and the bases 40 and 40c have the identical pitch and satisfy Equation (1) above. However, the markers 30 and 30c and the bases 40 and 40C do not need to satisfy Equation (1) with the identical pitch. Even in this case, as long as the width Wm of the markers 30 and 30c is larger than the width Wa of the protruding portion 52, a reduction in the visibility of the markers 30 and 30c due to the light streak (bokeh circle) may be suppressed even when the light is reflected by the recessed and protruding portion 55 of the resin layer 50 covering the markers 30 and 30c. The markers 30 and 30c and the bases 40 and 40C preferably satisfy Equation (1).

[Modification 5]

According to the first embodiment and the second embodiment, in the markers 30 and 30c and the resin layer 50, the width Wm of the markers 30 and 30c is smaller than twice the width Wa of the protruding portion 52. However, in the markers 30 and 30c and the resin layer 50, the width Wm of the markers 30 and 30c may be larger than twice the width Wa of the protruding portion 52. Even in this case, as long as the width Wm of the markers 30 and 30c is larger than the width Wa of the protruding portion 52, a reduction in the visibility of the markers 30 and 30c due to the light streak (bokeh circle) may be suppressed even when the light is reflected by the recessed and protruding portion 55 of the resin layer 50 covering the markers 30 and 30c. In the markers 30 and 30c and the resin layer 50, the width Wm of the markers 30 and 30c is preferably smaller than twice the width Wa of the protruding portion 52 and satisfies Equation (2).

[Modification 6]

According to the first embodiment and the second embodiment, the surface area of the markers 30 and 30c is 35% or more of the surface area of the bases 40 and 40c. However, the surface area of the markers 30 and 30c may be less than 35% of the surface area of the bases 40 and 40c. Even in this case, as long as the width Wm of the markers 30 and 30c is larger than the width Wa of the protruding portion 52, a reduction in the visibility of the markers 30 and 30c may be suppressed as described above. The surface area of the markers 30 and 30c is preferably 35% or less of the surface area of the bases 40 and 40c and satisfy Equation (3).

[Modification 7]

According to the first embodiment and the second embodiment, the lightness of the marker 30 is lower than that of the base 40. However, the lightness of the marker 30 may be higher than that of the base 40. Even in this case, it is possible to visually recognize the marker 30 by the contrast between the marker 30 and the base 40.

[Modification 8]

According to the first embodiment and the second embodiment, the width Wm of the markers 30 and 30c, the width Wb of the bases 40 and 40c, and the width Wa of the protruding portion 52 are the average values of the widths included in the range of 20 mm of the main body portion 10. That is, the visibility of the markers 30 and 30c may be improved as long as the width Wm of the markers 30 and 30c, the width Wb of the bases 40 and 40c, and the width Wa of the protruding portion 52 satisfy Equations (1) to (3) above as the average values in the range of 20 mm of the main body portion 10 even though they do not partially satisfy Equations (1) to (3).

[Modification 9]

In the guide wires 1 and 1C according to the first embodiment and the second embodiment, the distal end includes the coil body 60. However, the guide wires 1 and 1C may omit the coil body 60.
The present aspect has been described above based on the embodiments and modifications, but the embodiments described above as aspects are provided to facilitate understanding of the present aspect and not to limit the present aspect. The present aspect may be altered or modified without departing from the spirit thereof and the scope of claims, and the present aspect includes the equivalents thereof. The technical features may be deleted as appropriate unless the description indicates that they are essential.

DESCRIPTION OF REFERENCE NUMERALS

1, 1A to 1C Guide wire
2 Endoscope
10 Main body portion
20 Core shaft
21 Opening
22 Light emitting portion
23 Image sensor
30, 30a to 30c Marker
40, 40a to 40c Base
50 Resin layer
51 Recessed portion
52 Protruding portion
55 Recessed and protruding portion
60 Coil body
70 Distal end joint part
80 Proximal end side joint part

**Claims**

1. A guide wire comprising:

   a main body portion having an elongated outer shape and having a base and a linear marker alternately presented on an outer surface along an extending direction; and
   a resin layer having light permeability, covering the outer surface of the main body portion, and including a recessed and protruding portion where a recessed portion and a protruding portion are alternately formed along the extending direction of the main body portion, wherein
   in longitudinal cross-section along the extending direction of the main body portion, a width of the marker is wider than a width of the protruding portion.

2. The guide wire according to claim 1, wherein a pitch of the marker is different from a pitch of the protruding portion in the extending direction of the main body portion.

3. The guide wire according to claim 1 or 2, wherein the width of the marker is equal to or less than twice the width of the protruding portion in the longitudinal cross-section along the extending direction of the main body portion.

**EP 4 108 283 A1**

4. The guide wire according to any one of claims 1 to 3, wherein, on the outer surface of the main body portion, a surface area of the marker in a range of 20 mm along the extending direction of the main body portion is 35% or less of a surface area of the base.

5. The guide wire according to any one of claims 1 to 4, wherein, on the outer surface of the main body portion, lightness of the marker is lower than lightness of the base.

6. The guide wire according to any one of claims 1 to 5, wherein, on the outer surface of the main body portion, a boundary portion between the base and the marker is formed to be flat.

# FIG.1

FIG.2

# FIG.3

# FIG.4

# FIG.5

FIG.6

FIG.7

FIG.8

**FIG.9**

EP 4 108 283 A1

FIG.10

## FIG.11

## INTERNATIONAL SEARCH REPORT

| | International application No. |
|---|---|
| | PCT/JP2020/043585 |

**A. CLASSIFICATION OF SUBJECT MATTER**
Int. Cl. A61M25/09(2006.01)i
FI: A61M25/09 550

According to International Patent Classification (IPC) or to both national classification and IPC

**B. FIELDS SEARCHED**

Minimum documentation searched (classification system followed by classification symbols)
Int. Cl. A61M25/09

Documentation searched other than minimum documentation to the extent that such documents are included in the fields searched
Published examined utility model applications of Japan     1922-1996
Published unexamined utility model applications of Japan   1971-2021
Registered utility model specifications of Japan           1996-2021
Published registered utility model applications of Japan   1994-2021

Electronic data base consulted during the international search (name of data base and, where practicable, search terms used)

**C. DOCUMENTS CONSIDERED TO BE RELEVANT**

| Category* | Citation of document, with indication, where appropriate, of the relevant passages | Relevant to claim No. |
|---|---|---|
| A | JP 2008-264498 A (TERUMO CORP.) 06 November 2008, entire text, all drawings | 1-6 |
| A | JP 2010-35924 A (TERUMO CORP.) 18 February 2010, entire text, all drawings | 1-6 |
| A | JP 2003-93516 A (OLYMPUS OPTICAL CO., LTD.) 02 April 2003, entire text, all drawings | 1-6 |
| A | WO 2019/155828 A1 (GUNZE LTD.) 15 August 2019, entire text, all drawings | 1-6 |
| A | US 2009/0211909 A1 (K & L GATES LLP) 27 August 2009, entire text, all drawings | 1-6 |

☐ Further documents are listed in the continuation of Box C.  ☒ See patent family annex.

| * | Special categories of cited documents: | "T" | later document published after the international filing date or priority date and not in conflict with the application but cited to understand the principle or theory underlying the invention |
|---|---|---|---|
| "A" | document defining the general state of the art which is not considered to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
|---|---|
| 06.01.2021 | 19.01.2021 |

| Name and mailing address of the ISA/ | Authorized officer |
|---|---|
| Japan Patent Office | |
| 3-4-3, Kasumigaseki, Chiyoda-ku, | |
| Tokyo 100-8915, Japan | Telephone No. |

Form PCT/ISA/210 (second sheet) (January 2015)

| | International application No. |
|---|---|
| | PCT/JP2020/043585 |

| Patent Documents referred to in the Report | Publication Date | Patent Family | Publication Date |
|---|---|---|---|
| JP 2008-264498 A | 06.11.2008 | US 2008/0228109 A1 entire text, all drawings EP 1970092 A1 | |
| JP 2010-35924 A | 18.02.2010 | (Family: none) | |
| JP 2003-93516 A | 02.04.2003 | (Family: none) | |
| WO 2019/155828 A1 | 15.08.2019 | (Family: none) | |
| US 2009/0211909 A1 | 27.08.2009 | WO 2010/005623 A1 | |

Form PCT/ISA/210 (patent family annex) (January 2015)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 5619426 B
- JP 5509276 B
- WO 2009004876 A
- JP 2003275323 A